# EUROPÄISCHE PATENTSCHRIFT

(11) **EP 3 000 440 B1**
(45) Veröffentlichungstag und Bekanntmachung des Hinweises auf die Patenterteilung: **17.05.2017**
(21) Anmeldenummer: 15190710.2
(22) Anmeldetag: 21.08.2009
(51) Int. Cl.: A61F 2/28, A61F 2/36, A61F 2/30

(54) **ENDOPROTHESE MIT EINER STECKVERBINDUNG UND VERBESSERTER DREHSICHERUNG**
ENDOPROSTHESIS HAVING A PLUG-IN CONNECTION AND IMPROVED ROTATION PREVENTION
ENDOPROTHESE COMPRENANT UNE LIAISON D'ENFICHAGE ET UNE SECURITE CONTRE LA ROTATION AMELIOREE

(30) Priorität: 22.08.2008 DE 202008011178 U
(43) Veröffentlichungstag der Anmeldung: 30.03.2016
(62) Teilanmeldung aus: 09778045.6
(73) Patentinhaber: Waldemar Link GmbH & Co. KG, 22339 Hamburg (DE)
(72) Erfinder: Link, Helmut D., 22397 Hamburg (DE)
(74) Vertreter: Glawe, Delfs, Moll

(56) Entgegenhaltungen:
- EP-A- 0 474 015
- EP-A- 1 088 531
- WO-A-2007/028832
- DE-U1-202004 019 264
- US-A1- 2004 193 268

## Beschreibung

Die Erfindung betrifft eine Endoprothese, insbesondere zum mindestens teilweisen Ersatz eines Röhrenknochens, umfassend eine Steckverbindung zum Verbinden eines Schafts mit einem anderen Prothesenteil, wobei die Steckverbindung einen axialen Vorsprung und eine Aufnahme umfasst. Endoprothesen, insbesondere zur Versorgung von großräumigen Knochendefekten, sind üblicherweise mehrteilig ausgeführt und weisen einen schaftartigen langgestreckten Prothesenteil auf. An seinem Ende sind Einrichtungen angeordnet, die eine Verbindung mit anderen Prothesenteilen oder mit einem verbleibenden natürlichen Knochenabschnitt gestatten. Hierbei ist zu berücksichtigen, dass insbesondere bei Endoprothesen zum langstreckigen Knochenersatz, wie insbesondere für den Femur, große Kräfte aufgrund von Hebelwirkungen an den Verbindungsstellen auftreten. Gleiches gilt für Verbindungsstellen zwischen Segmenten der Prothese. Die dort eingesetzten Verbindungen müssen daher erheblichen Belastungen standhalten. Sie müssen nicht nur große Kräfte über lange Zeiträume sicher übertragen, sondern auch praktisch spielfrei sein und überdies bei Bedarf wieder leicht trennbar sein, um einen Austausch von Segmenten zu ermöglichen.

Es ist bekannt, mehrteilige Prothesen durch eine Steckverbindung mit einem Konus zu verbinden (DE 202004019264 U1). Um einen Schutz vor ungewünschter Verdrehung zu schaffen, ist eine gesonderte Verdrehsicherung mittels axial vorspringender Drehsicherungszapfen vorgesehen. Die Verbindung wird während der Operation hergestellt, in dem die Konussteckverbindung mit einem ausreichend großen Kraftaufwand ineinander gesteckt wird. Es hat sich aber gezeigt, dass die zur Erreichung einer sicheren Verbindung erforderliche Kraft nicht immer unter allen Umständen aufgebracht werden kann. Dies gilt insbesondere bei ungünstigen intraoperativen Umständen, wie einer schlechten Zugänglichkeit der Operationsstelle. Es ist daher bekannt, zur Sicherung des Konus auf Sicherungseinrichtungen zurückzugreifen. Dies geschieht in der Regel über eine Sicherungsschraube, die bei einer ersten bekannten Ausführungsform achsparallel zur Achse des Konus angeordnet ist, und die zu verbindenden Komponenten so zusammenzieht. Eine zweite Ausführungsform sieht im Wesentlichen vor, dass durch eine quer angeordnete Schraube auf die Flanke des Konus eingewirkt wird (DE 202005014269 U1; EP 1088531 A1). In beiden Fällen verkompliziert sich die Konstruktion der Prothesenteile, und es kommt zu einer Schwächung ausgerechnet in jenem Bereich, in dem es auf große Kraftübertragungsfähigkeit ankommt. Die Merkmale des Oberbegriffs des Anspruchs 1 sind aus EP0474015A1 bekannt.

Der Erfindung liegt die Aufgabe zugrunde, für eine Endoprothese der erstgenannten Art eine verbesserte Sicherung der Steckverbindung bereitzustellen.

Die erfindungsgemäße Lösung liegt in einer Endoprothese mit den Merkmalen des unabhängigen Anspruchs. Vorteilhafte Weiterbildungen sind Gegenstand der abhängigen Ansprüche.

Bei einer derartigen Endoprothese, insbesondere zum mindestens teilweisen Ersatz eines Röhrenknochens, umfassend eine Steckverbindung zum Verbinden eines Schafts mit einem anderen Prothesenteil, wobei die Steckverbindung einen axialen Vorsprung und eine Aufnahme umfasst, ist erfindungsgemäß vorgesehen, dass an dem einen Teil der Steckverbindung ein den Vorsprung hintergreifender Transversalkanal vorgesehen ist, dessen Mittelachse einen Versatz zur Achse einer an dem anderen Teil der Steckverbindung angeordneten Transversalbohrung aufweist, und einem in den Transversalkanal einzusetzenden Spannelement, dessen Spitze im eingesteckten Zustand in die Querbohrung eingreift. Unter den Vorsprung hintergreifend wird hier eine Position schaftseitig des Vorsprungs, beispielsweise an einem Bund des Vorsprungs, verstanden. Unter einem Versatz wird ein orientierter Abstand zwischen der Achse des Transversalkanals und der Transversalbohrung verstanden, der sich bei einem vollständig in die Aufnahme eingestecktem Vorsprung konstruktiv mit Nennmaßen ergibt. Die Orientierung des Abstandswerts ist dabei so, dass bei noch nicht vollständig eingeschobenem Vorsprung in die Aufnahme der Steckverbindung der Versatz einen höheren Wert aufweist, welcher sich vermindert, bis der Vorsprung schließlich vollständig in der Aufnahme aufgenommen ist. Wesentlich hierbei ist, dass der Versatz auch bei vollständig eingesetztem Vorsprung positiv bleibt; er soll weder null werden noch gar negativ sein.

Die Erfindung beruht auf der Erkenntnis, dass mit dem Spannelement in dem Transversalkanal durch Einwirken auf die Transversalbohrung eine den Vorsprung in Richtung Aufnahme spannende Reaktionskraft ausgeübt werden kann, wobei dank des beanspruchten positiven Achsversatzes die gewünschte spannende Wirkung auch bei Abweichung vom Nennmaß im Rahmen der Toleranz erreicht wird, und zwar auch bei ungünstiger Toleranzpaarung. Damit wird auch unter ungünstigen Umständen ein sicheres Spannen ermöglicht. Gerade die für derartige Steckverbindungen häufig verwendeten Konusverbindungen haben die Eigenart, dass auch bei nur geringen Toleranzen im Konusdurchmesser sich wegen des für die Selbsthemmung erforderlichen geringen Konuswinkels beträchtliche Differenzen in Bezug auf die Einstecktiefe ergeben. Wegen dieser Unterschiede konnte keine ausreichende Sicherheit durch eine einfache in eine Querbohrung eingesetzte Madenschraube erreicht werden; gegebenenfalls führte dies sogar zu einer Rückwärtsbewegung und damit zu einem Lösen der Konusverbindung. Die Erfindung hat nun erkannt, dass dieses aufgrund von unvermeidlichen Toleranzen immanente Problem der Steckverbindung auf elegante Weise dadurch gelöst werden kann, dass der Transversalkanal und die Transversalbohrung einen positiven Achsversatz aufweisen. Damit ist sichergestellt, dass auch bei ungünstigen Toleranzen die gewünschte Spannwirkung erreicht ist.

Der Versatz ist zweckmäßigerweise so gering gewählt, dass auch bei minimaler Einstecktiefe des Vorsprungs in die Aufnahme der Transversalkanal die Transversalbohrung überlappt. Damit ist Sicherheit auch für den Fall gegeben, dass bei Übertoleranz der Vorsprung nur schwer beziehungsweise gar nicht vollständig in die Aufnahme einzuschieben ist. Es hat sich bewährt, dass der Achsversatz mindestens 0,3 mm beträgt und nicht größer ist als 1,2 mm, vorzugsweise beträgt er zwischen 0,5 mm und 0,8 mm.

Für den Durchmesser genauer gesagt den Kerndurchmesser des Transversalkanals gilt, dass er mit Vorteil mindestens zwei-, vorzugsweise dreimal so groß ist wie die Differenz zwischen der maximalen und minimalen Einstecktiefe der Steckverbindung. Damit ist sichergestellt, dass unabhängig von der tatsächlichen Einstecktiefe eine ausreichende Überlappung zwischen dem Transversalkanal und der Transversalbohrung gegeben ist, so dass die erfindungsgemäße Spannwirkung noch realisiert werden kann. Mit Vorteil ist die Transversalbohrung so ausgeführt, dass sie konisch zulaufend ist. Im Regelfall wird sie als Sackbohrung ausgeführt sein, jedoch ist dies nicht zwingend erforderlich. Die Konizität ist verhältnismäßig groß und beträgt vorzugsweise über 30°, vorzugsweise liegt sie zwischen 45 und 75°. Mit dieser Konizität wird erreicht, dass durch den Eingriff des Spannelements in die Transversalbohrung jenes auf der zu dem anderen Teil der Steckverbindung gewandten Seite der Konizität zu liegen kommt. Beim weiteren Einbringen des Spannelements wird der Vorsprung weiter in die Aufnahme hineingetrieben.

Es hat sich bewährt, das Spannelement an seiner Spitze mit einer Verdrängungsfläche auszubilden, die vorzugsweise als Keilspitze ausgebildet ist. Weist das Spannelement selber eine Konizität auf, ist es unabhängig von einer eventuellen Konizität der Transversalbohrung oder es wird - bei deren Vorhandensein - deren Wirkung noch verstärkt.

In den meisten Fällen wird es genügen, wenn lediglich ein Transversalkanal mit achsversetzter Transversalbohrung angeordnet ist. Es kann aber auch vorgesehen sein, zwei oder mehr vorzusehen. Dies bietet sich insbesondere dann an, wenn der Transversalkanal in einem Drehsicherungszapfen angeordnet ist und in einem meist diametral gegenüberliegenden weiteren Drehsicherungszapfen ebenfalls ein solcher Transversalkanal angeordnet ist. Das Vorsehen von zweien (oder mehreren) bietet den Vorteil einer höheren Befestigungssicherheit. Allerdings kann das Risiko bestehen, dass eine statische Überbestimmtheit eintritt, wodurch es gegebenenfalls zu Verspannungen oder Überbelastungen kommen kann. Um dies zu vermeiden, ist erfindungsgemäss vorgesehen, dass mindestens ein Spannelement, vorzugsweise das zweite (oder weitere) Spannelement, eine elastische Spitze aufweist. Damit wird die Gefahr von Verspannungen beim Anziehen vermieden, und es wird die gewünschte zusätzliche Befestigungssicherheit erreicht. Ein Vorteil besteht darin, dass beim Ausfall des ersten Spannelements noch eine Sicherung erreicht wird durch das Spannelement mit der elastischen Spitze. Zwar kann es sein, dass hierbei ein gewisses leichtes Spiel entsteht, jedoch bietet dies den Vorteil, dass es dem Patienten wie auch dem betreuenden Arzt den Defekt des ersten Spannelements signalisiert, ohne dass deswegen gleich ein Totalausfall der Sicherungswirkung zu besorgen ist.

Mit Vorteil ist die Elastizität der Spitze durch eine Ummantelung realisiert, beispielsweise aus einem elastischen Material wie Gummi oder einem anderen elastischen Material mit hoher Bioverträglichkeit, beispielsweise bestimmte Arten von Polyethylen.

Die Erfindung wird nachfolgend in Bezug auf die beigefügte Zeichnung näher erläutert, in der ein vorteilhaftes Ausführungsbeispiel dargestellt ist. Es zeigen:
- Fig. 1: eine perspektivische Ansicht einer Endoprothese gemäß einem Ausführungsbeispiel der Erfindung;
- Fig. 2: eine vergrößerte Teilansicht einer Steckverbindung zweier Segmente der Endoprothese gemäß Fig. 1;
- Fig. 3a, b: Ausführungsbeispiele für Spannelemente;
- Fig. 4: eine Teilquerschnittansicht darstellend einen Achsversatz in einer Normallage;
- Fig. 5: eine Teilquerschnittansicht darstellend den Achsversatz in einer Maximallage und
- Fig. 6: eine Teilquerschnittansicht darstellend den Achsversatz in einer Minimallage.

Die Erfindung ist dargestellt am Beispiel einer Endoprothese, welche zum teilweisen Ersatz des Femurknochens im Bereich seines oberen Endes ausgebildet ist. Die Prothese besteht aus zwei Segmenten, einem oberen Prothesenteil 1 mit einem Gelenkzapfen 10 zur Aufnahme eines Kugelkopfs (nicht dargestellt) als Teil eines künstlichen Hüftgelenks und einem Schaft 2, welcher in einer Knochenmarkhöhlung des verbleibenden Teils des natürlichen Femurs einzusetzen ist. Der Prothesenteil 1 und der Schaft 2 sind über eine Steckverbindung 3 miteinander verbunden. An der Steckverbindung ist ferner noch eine gesonderte Drehsicherung 4 vorgesehen.

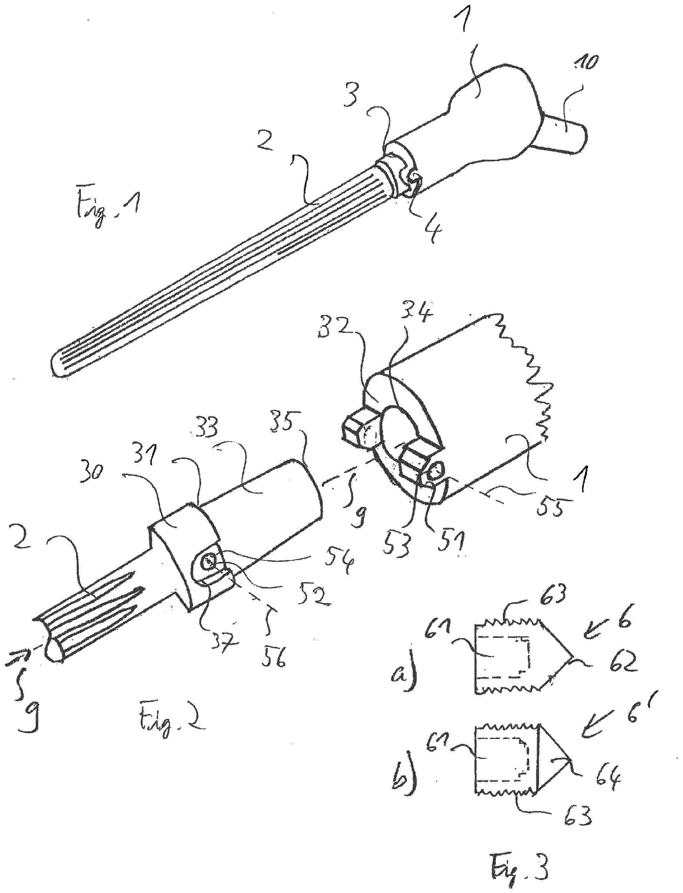

Nachfolgend werden die Steckverbindung 3 und die Drehsicherung 4 näher unter Bezugnahme auf Fig. 2 erläutert. Man erkennt im linken Bildteil das obere Ende des Schafts 2. Dort ist ein verdickter Bund 30 mit einer nach außen weisenden Stirnfläche 31 angeordnet. Auf dieser erstreckt sich ein auf der Axialverlängerung der Achse des Schafts 2 angeordneter konusartiger Vorsprung 33. Der Konus ist an seinem äußeren Ende 35 stumpfartig abgeschnitten. Weiter sind an der Radialfläche des Bunds 30 taschenartige Ausnehmungen 37 ausgebildet, die sich von der Stirnfläche 31 zum Schaft 2 hin erstrecken. Sie weisen eine zur Mittelachse des Schafts 2 gerichtete Querbohrung 52 auf. Sie ist als ein Sackloch ausgeführt mit einer Konizität 54 an seinem Grund.

In der rechten Bildhälfte von Fig. 2 ist das untere Ende des den Femurkopf bildenden Prothesenteils 1 dargestellt. Es ist von generell zylinderförmiger Gestalt und weist eine Stirnfläche 32 an seinem unteren Ende auf. In diese ist zentrisch eine Aufnahmebohrung 34 angeordnet, deren Innenwandung konisch nach innen geformt ist. Die Abmessungen der Aufnahmebohrung 34 sind so auf den Vorsprung 33 des Schaftteils 2 abgestimmt, dass der Vorsprung 33 nahezu vollständig in die Aufnahmebohrung 34 eingeschoben werden kann, bis zwischen den Stirnflächen 31 und 32 nur noch ein geringer Freiraum (typisch sind Werte von 0,75 mm) verbleibt. In jedem Fall ist die Tiefe der Aufnahmebohrung 34 so bemessen, dass die Stirnflächen 31, 32 aufeinander zu liegen kommen, bevor die Spitze 35 des Vorsprungs am Grund der Aufnahme 34 zu liegen kommt. Die aufgrund der Abmessungen konstruktiv vorgegebene Entfernung zwischen dem äußeren Ende 35 und der Stirnseite 32 bildet ein Einstecktiefenmaß.

Weiter sind an dem Prothesenteil 1 zwei diametral einander gegenüberliegende, axial vorspringende Drehsicherungszapfen 51 ausgebildet. Sie wirken im zusammengesetzten Zustand mit den entsprechenden Ausnehmungen 37 im Bund 30 des Schafts 2 zusammen. Die Breite der Drehsicherungszapfen 51 ist hierbei auf die Breite der Ausnehmungen 37 bestimmt, so dass sich - einschließlich eines zum leichten Einsetzen erforderlichen Spiels - eine Verdrehungssicherheit des Schafts 2 gegenüber dem Prothesenteil 1 ergibt.

In dem Drehsicherungszapfen ist quer zur Einsteckrichtung 9 ein Transversalkanal 53 angeordnet. Dieser weist ein Innengewinde auf, in welches ein Spannelement 6 eingeschraubt werden kann. Die Mittelachse 55 des Transversalkanals 53 schneidet die Einsteckrichtung 9 in einem rechten Winkel. - Dementsprechend ist an der Radialfläche innerhalb der Ausnehmung 37 eine Transversalbohrung 52 ausgebildet. Sie ist als ein Sackloch mit einer Konizität 54 am Grund ausgebildet. Die Spitze der Konizität definiert eine Mittelachse 56. Es sei angemerkt, dass die Konizität nicht unbedingt voll zu sein braucht, sondern auch stumpf abgeschnitten sein kann; an der Lage der Mittelachse 56 ändert dies nichts.

Das Spannelement 6 ist in Fig. 3 dargestellt. Es ist ähnlich einer Madenschraube ausgebildet mit einem zylinderförmigen Grundkörper, dessen Mantelfläche ein Außengewinde 63 trägt. Dies ist so bemessen, dass es in das Innengewinde des Transversalkanals 53 eingreift. An einer Stirnseite ist eine hexagonale Vertiefung 61 angeordnet, die als Aufnahme für einen Schraubenschlüssel als Betätigungswerkzeug dient. Die gegenüberliegende Stirnseite 62 ist spitzkegelig ausgeformt. Mit Vorteil ist der Kegelwinkel so bemessen, dass er mit dem die Konizität 54 der Transversalbohrung 52 bestimmenden Konuswinkel übereinstimmt.

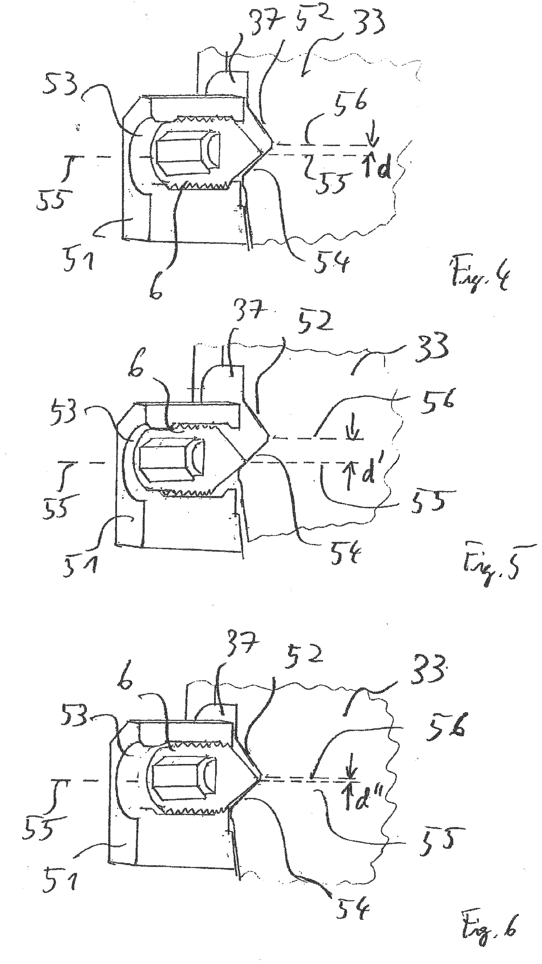

Das Zusammenwirken des Transversalkanals 53 mit der Transversalbohrung 52 wird unter Bezugnahme auf die Figuren 4 bis 6 näher erläutert. Dargestellt ist ein Teilquerschnitt durch die Steckverbindung im eingesteckten Zustand, d.h. der Vorsprung 33 befindet sich in der Aufnahme 34. Er sei soweit eingeschoben, bis ein fester Sitz aufgrund der konischen Form erreicht ist. Im dargestellten Ausführungsbeispiel seien die Anmessungen so gewählt, dass sich ein restliches Spiel zwischen den Stirnflächen 31, 32 von 1,25 mm ergibt. Erfindungsgemäß ist der Achsversatz d zwischen der Mittelachse 55 des Transversalkanals 53 und der Mittelachse 56 der Transversalbohrung 52 so gewählt, dass sich bei dieser Lage der Steckverbindung - die als Normallage definiert ist - ein Achsversatz von 0,75 mm ergibt. Damit wird erreicht, dass bei in dem Transversalkanal 53 eingeschraubten Spannelement 6 dieses mit einem Zentrum an der der Stirnfläche 31 zugewandten Flanke des konischen Teils der Transversalbohrung 52 zu liegen kommt. Durch Eindrehen des Spannelements 6 wird damit eine den Vorsprung 33 weiter in die Aufnahme 34 treibende Kraft (in Fig. 4 bis 6 nach unten) auf den Vorsprung 33 ausgeübt. Die Steckverbindung wird dadurch gespannt.

Aufgrund von Toleranzen bei der Herstellung und/oder nicht vollständig korrektem Einsetzen können sich Toleranzen in Bezug auf die Lage der Prothesenteile 1, 2 ergeben. Für den Fall einer ungünstigen Toleranzpaarung, nämlich Vorsprung 33 an oberer Toleranzgrenze und Aufnahme 34 an unterer Toleranzgrenze, oder nicht vollständigem Einstecken durch den Operateur ergibt sich die in Fig. 5 dargestellte Situation. Die Einstecktiefe der Steckverbindung 3 ist hier geringer, so dass sich ein Spiel zwischen den Stirnflächen 31, 32 von 1,7 mm ergibt. Der tatsächliche Achsversatz d' beträgt dann 1,2 mm. Der Durchmesser der Transversalbohrung 52 ist unter Berücksichtigung des Achsversatzes hierbei so gewählt, dass die Mittellinie 55 noch in dem Bereich der der Stirnseite 31 zugewandten Flanke zu liegen kommt. Damit kommt beim Einschieben des Spannelements 6 dieses mit seiner Spitze in Eingriff mit dieser Flanke, wodurch die gewünschte Spannwirkung erreicht wird. Es ergibt sich auch in diesem Fall bei ungün-stiger Toleranzpaarung beziehungsweise nicht vollständigen Einsetzens der gewünschte spielfreie Sitz.

Der entgegengesetzte Fall einer Toleranzpaarung, nämlich mit einem Vorsprung 33 an der unteren Toleranzgrenze und einer Aufnahme 34 an der oberen Toleranzgrenze, ist in Fig. 6 dargestellt. Es ergibt sich dabei eine Einstecktiefe der Steckverbindung, die größer ist als diejenige, wie sie konstruktiv für die Normallage (siehe Fig. 4) vorgesehen ist. Durch die erfindungsgemäße Wahl des Achsversatzes ist sichergestellt, dass auch in diesem Fall bei einem Spiel von 0,8 mm noch ein positiver tatsächlicher Achsversatz d'' von 0,3 mm besteht. Damit ist auch in diesem an sich sehr ungünstigen Fall der Toleranzpaarung noch ein sicheres Spannen gewährleistet.

In Fig. 3b ist noch eine Variante für das Spannelement 6' dargestellt. Es unterscheidet sich von dem in Fig. 3a dargestellten Spannelement 6 im Wesentlichen dadurch, dass an der Spitze 62 noch eine Ummantelung 64 aus elastischem Material angeordnet ist. Bei dem elastischen Material kann es sich um ein gummiartiges Material oder um gut bioverträgliches, hochelastisches Polyethylen handeln. Zur Erhöhung der Elastizität kann auch die gesamte Spitze 62' aus dem elastischem Material bestehen. Eine derartige Ausbildung des Spannelements 6' ist von Vorteil, wenn noch in einem zweiten (oder weiteren) Drehsicherungszapfen ein entsprechender Transversalkanal 53' (siehe gestrichelte Linie in Fig. 2) eine entsprechende Transversalbohrung in der zugeordneten Aussparung (nicht dargestellt) vorgesehen ist. Mit der elastischen Gestaltung an der Spitze wird durch das Spannelement 6' erreicht, dass der Gefahr einer statischen Überbestimmtheit und damit einer Überlastung beziehungsweise verschlechterten Positionierung wirkungsvoll begegnet wird. Mit Vorteil ist der Achsversatz des zweiten Transversalkanals 53' größer bemessen als derjenige des ersten Transversalkanals 53. Dies bietet den Vorteil, dass dabei eine Rückfallposition zur Verfügung steht, bei der ein zwar geringes und damit fühlbares, aber nicht kritisches Spiel sich einstellt.

## Patentansprüche

1. Prothese, insbesondere zum mindestens teilweisen Ersatz eines Röhrenknochens, umfassend eine Steckverbindung zum Verbinden eines Schafts (2) mit einem anderen Prothesenteil (1), wobei die Steckverbindung (3) einen axialen Vorsprung (33) und eine Aufnahme (34) umfasst, wobei an dem einen Teil der Steckverbindung (3) ein die Steckverbindung hintergreifender radialer Transversalkanal (53) vorgesehen ist, dessen Mittelachse (55) einen Versatz (d) zur Achse (56) einer an dem anderen Teil der Steckverbindung angeordneten Transversalbohrung (52) aufweist, und wobei die Prothese ein in den Transversalkanal (53) einzusetzendes Spannelement (6) aufweist, dessen Spitze (62) im eingesetzten Zustand in die Transversalbohrung (52) eingreift, **dadurch gekennzeichnet, dass** die Spitze des Spannelements (6, 6') elastisch ausgebildet ist.

2. Prothese nach Anspruch 1, **dadurch gekennzeichnet, dass** der Achsabstand mindestens so groß gewählt ist, dass er auch bei maximaler Einstecktiefe positiv ist.

3. Prothese nach Anspruch 1 oder 2, **dadurch gekennzeichnet, dass** der Achsversatz höchstens so groß gewählt ist, dass auch bei minimaler Einstecktiefe der Transversalkanal (53) die Transversalbohrung (52) überlappt.

4. Prothese nach einem der vorangehenden Ansprüche, **dadurch gekennzeichnet, dass** der Achsversatz mindestens 0,3 mm, vorzugsweise 0,5 mm beträgt.

5. Prothese nach einem der vorhergehenden Ansprüche, **dadurch gekennzeichnet, dass** ein Kerndurchmesser des Transversalkanals (53) mindestens zweimal, vorzugsweise dreimal so groß ist wie die Differenz zwischen minimaler und maximaler Einstecktiefe.

6. Prothese nach einem der vorangehenden Ansprüche, **dadurch gekennzeichnet, dass** die Transversalbohrung (52) an ihrem Grund konisch zulaufend ist.

7. Prothese nach einem der vorhergehenden Ansprüche, **dadurch gekennzeichnet, dass** der Transversalkanal (53) in einem achsparallel vorspringenden Drehsicherungszapfen (51) der Steckverbindung angeordnet ist.

8. Prothese nach Anspruch 7, **dadurch gekennzeichnet, dass** ein zweiter Transversalkanal (53') in einem zweiten, vorzugsweise diametral gegenüber liegenden Drehsicherungszapfen (51') angeordnet ist.

9. Prothese nach Anspruch 8, **dadurch gekennzeichnet, dass** der zweite Transversalkanal (53') einen anderen, vorzugsweise größeren Achsabstand aufweist.

10. Prothese nach einem der vorhergehenden Ansprüche, **dadurch gekennzeichnet, dass** die Spitze (62) als eine vorzugsweise keilförmige Verdrängungsfläche ausgebildet ist.

11. Prothese nach einem der Ansprüche 1 bis 10, **dadurch gekennzeichnet, dass** die Spitze (62) mit einer Ummantelung (64) aus elastischem Material, vorzugsweise hochelastischem Polyethylenmaterial, ausgebildet ist.

## Claims

1. Prosthesis, particularly for the at least partial replacement of a long bone, comprising a plug-in connection for connecting a shaft (2) to another part (1) of the prosthesis, wherein the plug-in connection (3) comprises an axial projection (33) and a socket (34), wherein a radial transverse channel (53) is provided on one part of the plug-in connection (3), said radial transverse channel (53) engaging behind the plug-in connection and having a center axis (55) that is offset (d) relative to the axis (56) of a transverse bore (52) arranged on the other part of the plug-in connection, and wherein the prosthesis has a clamping element (6) which is to be inserted into the transverse channel (53) and whose point (62), in the inserted state, engages in the transverse bore (52), **characterized in that** the tip of the clamping element (6, 6') is elastic.

2. Prosthesis according to Claim 1, **characterized in that** the axial spacing chosen is at least so great that it is positive even at a maximum depth of insertion.

3. Prosthesis according to Claim 1 or 2, **characterized in that** the axial offset chosen is at most so great that the transverse channel (53) overlaps the transverse bore (52) even at a minimum depth of insertion.

4. Prosthesis according to one of the preceding claims, **characterized in that** the axial offset measures at least 0.3 mm, preferably 0.5 mm.

5. Prosthesis according to one of the preceding claims, **characterized in that** a core diameter of the transverse channel (53) is at least twice as great, preferably three times as great, as the difference between minimum and maximum depth of insertion.

6. Prosthesis according to one of the preceding claims, **characterized in that** the transverse bore (52) tapers conically at the bottom thereof.

7. Prosthesis according to one of the preceding claims, **characterized in that** the transverse channel (53) is arranged in an axially parallel projecting anti-rotation stub (51) of the plug-in connection.

8. Prosthesis according to Claim 7, **characterized in that** a second transverse channel (53') is arranged in a second, preferably diametrically opposite anti-rotation stub (51').

9. Prosthesis according to Claim 8, **characterized in that** the second transverse channel (53') has another and preferably greater axial spacing.

10. Prosthesis according to one of the preceding claims, **characterized in that** the tip (62) is designed as a preferably wedge-shaped displacement surface.

11. Prosthesis according to one of Claims 1 to 10, **characterized in that** the tip (62) is designed with a covering (64) made of elastic material, preferably highly elastic polyethylene material.

## Revendications

1. Prothèse, en particulier pour le remplacement au moins partiel d'un os long, comprenant une connexion par enfichage pour le raccordement d'une tige (2) à une autre partie de prothèse (1), la connexion par enfichage (3) comprenant une saillie axiale (33) et un logement (34), un canal transversal radial (53) venant en prise par l'arrière avec la connexion par enfichage étant prévu au niveau de l'une des parties de la connexion par enfichage (3), son axe médian (55) présentant un décalage (d) par rapport à l'axe (56) d'un alésage transversal (52) disposé au niveau de l'autre partie de la connexion par enfichage, et la prothèse présentant un élément de serrage (6) devant être inséré dans le canal transversal (53), dont la pointe (62), dans l'état inséré, vient en prise dans l'alésage transversal (52), **caractérisée en ce que** la pointe de l'élément de serrage (6, 6') est réalisée sous forme élastique.

2. Prothèse selon la revendication 1, **caractérisée en ce que** la distance entre les axes est choisie de manière à être au moins suffisante pour être positive même à la profondeur d'enfichage maximale.

3. Prothèse selon la revendication 1 ou 2, **caractérisée en ce que** le décalage entre les axes est choisi de manière à être au maximum tel que même à la profondeur d'enfichage minimale, le canal transversal (53) chevauche l'alésage transversal (52).

4. Prothèse selon l'une quelconque des revendications précédentes, **caractérisée en ce que** le décalage entre les axes vaut au moins 0,3 mm, de préférence 0,5 mm.

5. Prothèse selon l'une quelconque des revendications précédentes, **caractérisée en ce qu'**un diamètre de coeur du canal transversal (53) est au moins le double, de préférence le triple de la différence entre la profondeur d'enfichage minimale et maximale.

6. Prothèse selon l'une quelconque des revendications précédentes, **caractérisée en ce que** l'alésage transversal (52) est effilé sous forme conique au niveau de sa base.

7. Prothèse selon l'une quelconque des revendications précédentes, **caractérisée en ce que** le canal transversal (53) est disposé dans un tourillon de fixation en rotation (51) de la connexion par enfichage saillant avec son axe parallèle.

8. Prothèse selon la revendication 7, **caractérisée en ce qu'**un deuxième canal transversal (53') est disposé dans un deuxième tourillon de fixation en rotation (51') de préférence diamétralement opposé.

9. Prothèse selon la revendication 8, **caractérisée en ce que** le deuxième canal transversal (53') présente une autre distance entre les axes, de préférence plus grande.

10. Prothèse selon l'une quelconque des revendications précédentes, **caractérisée en ce que** la pointe (62) est réalisée sous forme de surface de déplacement de préférence en forme de coin.

11. Prothèse selon l'une quelconque des revendications 1 à 10, **caractérisée en ce que** la pointe (62) est réalisée avec une enveloppe (64) en matériau élastique, de préférence en matériau à base de polyéthylène hautement élastique.
